# EUROPEAN PATENT SPECIFICATION

(11) **EP 1 256 342 B1**
(45) Date of publication and mention of the grant of the patent: **11.01.2006**
(21) Application number: 00974960.7
(22) Date of filing: 13.11.2000
(51) Int. Cl.: A61K 31/315, A61K 31/555, A61K 31/44, A61K 31/381, A61K 31/351, A61P 31/10, C07F 3/06, C07D 213/79, C07D 213/55, C07D 213/80, C07D 277/06, C07D 309/40, C07D 213/89, C07D 333/40, C07D 295/20, C07D 277/04

(54) **HYPOGLYCEMICS COMPRISING ORGANIC ZINC (II) COMPLEXES**
HYPOGLYKÄMISCHE MITTEL, DIE ORGANISCHE ZINKKOMPLEXE ENTHALTEN
HYPOGLYCEMIANTS A BASE DE COMPLEXES DE ZINC (II) ORGANIQUES

(30) Priority: 30.11.1999 JP 34005899; 18.05.2000 JP 2000145849
(43) Date of publication of application: 13.11.2002
(73) Proprietor: Japan Science and Technology Agency, Kawaguchi-shi Saitama (JP)
(72) Inventor: KOJIMA, Yoshitane, Izumi-shi, Osaka 594-0013 (JP); SAKURAI, Hiromu, Nagaokakyo-shi, Kyoto 617-0825 (JP)
(74) Representative: Cresswell, Thomas Anthony
(86) International application number: PCT/JP2000/007991
(87) International publication number: WO 2001/039769

(56) References cited:
- EP-A- 1 026 163
- WO-A-95/24911
- WO-A-97/41856
- WO-A-99/22734
- WO-A1-87/01281
- FR-A- 2 686 603
- FR-A- 2 729 957
- JP-A- 9 255 678
- JP-A- 49 048 813
- US-A- 3 517 040
- US-A- 4 080 329
- US-A- 4 665 064
- US-A- 4 764 633
- US-A- 4 866 052
- BRIGGS, ELIZABETH M. ET AL: "Complexes of transition metals with oxygen donor ligands. I. Electronic and infrared spectra of some complexes of 2,6-diethyl-4-pyrone" JOURNAL OF THE CHEMICAL SOCIETY [SECTION] A: INORGANIC, PHYSICAL, THEORETICAL (1969), (12), 1835-9 , XP009013013
- DE JAGER, A. ET AL: "The coordination chemistry of 2,6-dimethyl-4-pyrone" INORGANICA CHIMICA ACTA (1976), 20(1), 59-64 , XP009012175
- CINGI, MARINA BIAGINI ET AL: "Chlorides of bivalent metals with 2,6-dimethyl-.gamma.-pyrone" GAZZETTA CHIMICA ITALIANA (1965), 95(5), 425-31 , XP009012184
- BROWN, C. J. ET AL: "Bis(2,6-dimethyl-4H-pyran-4-one)dinitrato zinc, [Zn(NO3)2(C7H8O2)2]" ACTA CRYSTALLOGRAPHICA, SECTION C: CRYSTAL STRUCTURE COMMUNICATIONS (1984), C40(3), 368-70 , XP009012146
- HOFFMULLER, WINFRIED ET AL: "Metal complexes of biologically important ligands. Part 119. A tetrahedra zinc complex (L-tert-leucine)2ZnCl2 of carboxylate coordinated L-tert-leucine in the zwitterionic form" ZEITSCHRIFT FUER NATURFORSCHUNG, B: CHEMICAL SCIENCES ( 1999 ), 54(6), 734-736 , XP009012172
- CORRADI, A. BONAMARTINI ET AL: "Coordination properties of N-p-tolylsulfonyl-L-glutamic acid toward metalII Part 1. Crystallographic study on ZnII and CdII complexes" POLYHEDRON ( 1999 ), 18(14), 1975-1982 , XP002246451
- JACKSON, RICHARD F. W. ET AL: "Concise Synthesis of Enantiomerically Pure Phenylalanine, Homophenylalanine, and Bishomophenylalanine Derivatives Using Organozinc Chemistry: NMR Studies of Amino Acid-Derived Organozinc Reagents" JOURNAL OF ORGANIC CHEMISTRY ( 1998 ), 63(22), 7875-7884 , XP002246452
- BELL P. ET AL.: "Preparation and Structure of Zinc Complexes of Cysteine Derivatives" ZEITSCHRIFT FÜR NATURFORSCHUNG, TEIL B: ANORGANISCHE CHEMIE, ORGANISCHE CHEMIE, vol. 39b, no. 12, 1984, pages 1732-1737, XP009013048
- HARIHARAN, MEENA ET AL: "Crystal structure of a complex of glycine with zinc chloride" ZEITSCHRIFT FUER KRISTALLOGRAPHIE ( 1989 ), 188(3-4), 217-22 , XP009012998
- SIVASANKAR, B. N. ET AL: "Thermal, spectral and magnetic studies on glycine complexes of cobalt(II) nickel(II) and zinc(II) with hydrazine" JOURNAL OF THERMAL ANALYSIS ( 1996 ), 46(1), 117-27 , XP009012996
- INOMATA ET AL.: "The metal complexes of amino acids and their N-substituted derivatives-VII. The IR spectra and normal coordinate analyses of bivalent metal complexes with N-methylglycine and N-phenylglycine" SPECTROCHIMICA ACTA, PART A: MOLECULAR AND BIOMOLECULAR SPECTROSCOPY, vol. 44a, no. 1, 1988, pages 97-107, XP009012997
- MARCHESELLI L ET AL: "SYNTHESIS CHARACTERIZATION AND EVALUATION OF BIOLOGICAL ACTIVITY OFPALLADIUM (II) AND PLATINUM (II) COMPLEXES WITH DITHIOCARBAMIC ACIDS AND THEIR DERIVATIVES AS LIGANDS" EUROPEAN JOURNAL OF MEDICINAL CHEMISTRY, EDITIONS SCIENTIFIQUE ELSEVIER, PARIS, FR, vol. 28, no. 4, 1993, pages 347-352, XP000907110 ISSN: 0223-5234
- SUBRAMANIAM S: "THE EMERGING ROLE OF THIAZOLIDINEDIONES IN THE TREATMENT OF DIABETES-MELLITUS AND RELATED DISORDERS" CLINICAL AND EXPERIMENTAL HYPERTENSION, MARCEL DEKKER, NEW YORK, NY, US, vol. 21, no. 1/2, 1999, pages 121-136, XP001028567 ISSN: 1064-1963
- CLARK D A ET AL: "SUBSTITUTED DIHYDROBENZOPYRAN AND DIHYDROBENZOFURAN THIAZOLIDINE-2,4-DIONES AS HYPOGLYCEMIC AGENTS" JOURNAL OF MEDICINAL CHEMISTRY, AMERICAN CHEMICAL SOCIETY. WASHINGTON, US, vol. 34, 1991, pages 319-325, XP001019083 ISSN: 0022-2623
- FRASER P J ET AL: "Carbene complexes of iridium, rhodium, manganese, chromium, and ironcntaining thiazolidinylidene and pyridinylidene ligands" JOURNAL OF THE CHEMICAL SOCIETY, DALTON TRANSACTIONS, CHEMICAL SOCIETY. LETCHWORTH, GB, 1974, pages 760-764, XP002191975 ISSN: 1472-7773
- NAGASE M ET AL: "SYNTHETIC, SPECTROSCOPIC ANFD X-RAY CRYSTALLOGRAPHIC STUDIES OF BIVALENT METAL COMPLEXES WITH AMINO ACIDS HAVING A THIAZOLIDINE RING II. PROPERTIES OF BIVALENT METAL COMPLEXES WITH RACEMIC 4-THIAZOLIDINECARBOXYLIC ACID" INORGANICA CHIMICA ACTA, LAUSANNE, CH, vol. 152, no. 4, 1988, pages 211-214, XP002936541 ISSN: 0020-1693
- LOULOUDI, M. ET AL: "Cobalt(II), nickel(II), copper(II) and zinc(II) complexes of thiazolidine" BULLETIN DES SOCIETES CHIMIQUES BELGES (1988), 97(2), 91-7 , XP009013248
- CHEN, BANG-LIN ET AL: "Thiophene-2,5-dicarboxylic acid incorporated self-assembly of one-, two- and three-dimensional coordination polymers" NEW JOURNAL OF CHEMISTRY (1999), 23(8), 877-883 , XP009013291
- VITTAL YACHANDRA ET AL.: 'X-ray absorption spectra and the coordination number of Zn and Co carbonic anhydrase as a function of pH and inhibitor binding' JOURNAL OF AMERICAN CHEMICAL SOCIETY vol. 105, no. 22, 1983, pages 6596 - 6604, XP002936539
- G. FARAGLIA ET AL.: 'The thermal behaviour of zinc and cadmium dihalide complexes with O- and S-donors' THERMOCHIMICA ACTA vol. 191, no. 1, 1991, pages 95 - 104, XP002936540
- MAKOTO NAGASE ET AL.: 'Synthetic, spectroscopic and X-ray crystallographic studies of bivalent metal complexes with amino acids having a thiazolidine ring. II. Properties of bivalent metal complexes with racemic 4-thiazolidinecarboxylic acid' INORGANIC CHIMICA ACTA vol. 152, no. 4, 1988, pages 211 - 214, XP002936541

## Description

### Technical Field

The present invention relates to a hypoglycemic agent comprising organic zinc (II) complexes. More particularly, the invention relates to a hypoglycemic agent comprising an organic zinc (II) complex having a pyridine ring-containing ligand. The invention also relates to an oral formulation comprising organic zinc (II) complexes which have a hypoglycemic effect and an insulin-like effect.

### Background of the Invention

In spite of a rapid advance of medical technologies, various adult diseases such as Alzheimer's disease, ischemic heart failure and diabetes are still remaining as substantial problems in Japan. Among these diseases, a diabetes is prevailing in a large number of latent patients, which is still increasing continuously year by year. Currently, the diabetes is defined as a disease which exhibits an abnormal glucose, protein or lipid metabolism due to an absolute or relative deficiency of insulin and which leads to a characteristic complication of the diabetes, such as a nephropathy, retinopathy, neuropathy and the like, resulting from a chronic hyperglycemia. The diabetes is classified by world Health Organization (WHO) broadly into two types, namely, type I (insulin-dependent) diabetes and type II (insulin-non-dependent) diabetes. In the type I diabetes due to an absolute deficiency of insulin, B cells in pancreas are destroyed due to an autoimmune islet inflammation, resulting in suppressed synthesis or secretion of the insulin. Accordingly, an insulin injection is the only therapy which is possible practically. Such insulin is painful for patients, and thus therapeutic agents as substitutes for the insulin are expected all over the world.

On the other hand, a type II diabetes involves a relative low sensitivity of an insulin to cells. Factors of the onset of the type II diabetes are thought due to obesity, stress or lack of exercise.

The reduced effect of the insulin in the diabetes leads to glucose consumption impairment in tissues inducing an energy production, which is compensated by a promoted neutral fat decomposition in fat cells, and resulting in a promoted release of free fatty acids (FFA) as an energy source. While the neutral fat decomposition is promoted by an activation of a hormone-sensitive lipase, this enzyme is activated by catecholamine, glucagon, adrenotropic hormone and the like but inactivated by insulin. In diabetes, the release of FFA from fat tissues is promoted not only by an insulin deficiency but also by an increased glucagon secretion.

In addition, the type I diabetes can currently be treated only by a subcutaneous injection of an insulin, and oral therapeutic agents as substitutes for the insulin are desired to be developed. One of such therapeutic agents is vanadyl sulfate which has already been employed in clinical trials in the United States. On the other hand, zinc (II) ion known to be less toxic when compared with vanadium had become to be known to have an insulin-like activity since about 1980 (L.Coulston and P.Dandona, "Insulin-like effect of zinc on adipocytes", Diabetes,29, 665-7(1980); J.M.May and C.S.Contoreggi, "The mechanism of the insulin-like effects of ionic zinc", J.Biol.Chem., 257, 4362-8(1982); A.Shisheva, D.Gefel and Y.Shechter, "Insulin-like effect of zinc ion in vitro and in vivo" 'Zn²⁺ is the first agent other than vanadate that on oral administration is able to restore tissue ability to metabolism glucose), Diabetes, 41, 982-8 (1992)).

Since vanadyl sulfate and zinc (II) ion (zinc sulfate or zinc chloride) are inorganic salts, they have difficulties in penetrating biological membranes and being incorporated into tissues. For the purpose of overcoming such difficulties, a hypoglycemic agent, which is less toxic than vanadium, which exhibited appropriate degrees of a stability and a fat-soluble insulin-like effect, and which is more effective than vanadyl complexes, is desired to be developed.

### Disclosure of the Invention

The present invention provides hypoglycemic agents which are less toxic when compared with vanadium, which exhibit appropriate degrees of stabilities and fat-soluble insulin-like effects. The invention also provides a hypoglycemic agent capable of being administered orally and having an insulin-like effect.

We made an extensive study on zinc (II) compounds, and finally discovered that organic zinc (II) complexes, especially organic zinc (II) complexes having a ligand of a pyridine ring-containing compound, are less toxic when compared with those of corresponding vanadium complexes and exhibit appropriate degrees of stabilities and fat-soluble insulin-like effects, and that such organic zinc (II) complexes can be administered orally.

Accordingly, the invention relates to hypoglycemic agents which comprise organic zinc (II) complexes, which have hypoglycemic effect and insulin-like effect and thus they are useful as a prophylactic or therapeutic agent against the diabetes.

More particularly, the invention relates to organic zinc (II) complexes having a pyridine ring-containing ligand. For use is a method of treatment on the human or animal body.

The invention further relates to the use of such an organic zinc (II) complex in the manufacture of a medicament for the treatment of diabetes.

The invention also relates to such a use which involves an oral formulation comprising organic zinc (II) complexes. Moreover, the invention relates to such a use wherein the oral formulation comprises hypoglycemic agents which have hypoglycemic effect and insulin-like effect and thus they are useful as a prophylactic or therapeutic agent against the diabetes.

Moreover, the invention relates to organic zinc (II) complexes for use in producing a hypoglycemic agent useful for a prophylactic or therapeutic agent against the diabetes. The invention relates the use of a therapeutically effective amount of organic zinc (II) complexes in the manufacture of a medicament for preventing or treating a hyperglycemic disease such as diabetes

While an inorganic zinc (II) ion compound has already been known to have an insulin-like effect, its unsatisfactory efficacy and difficulty in penetrating a biological membrane to be incorporated into an in vivo tissue makes it difficult to exhibit any in vivo effect.

We made an extensive study on a zinc (II) which can penetrate a biological membrane and can readily be incorporated into an in vivo tissue, and finally discovered that organic zinc (II) complexes have each sterically bulky organic moiety and zinc (II) as its center metal which form structures allowing them to penetrate biological membranes and to be incorporated readily into in vivo tissues and also have sufficient insulin-like effect and hypoglycemic effect.

### Brief Description of the Drawings

Figure 1 shows the effect of an inventive organic zinc (II) complex on the release of a fatty acid from fat cells. In Figure 1, 1 denotes a blank, 2 denotes a control, 3 to 5 denote positive controls and 6 to 14 denote inventive compounds.
Figure 2 shows the blood glucose curves observed in a glucose tolerance test in KK-A^{y} mice treated with or without an inventive organic zinc (II) complex.
Figure 3 shows the blood glucose curves observed in a glucose tolerance test in humans (diabetes patients and normal healthy volunteers).
Figure 4 shows the change in the blood glucose after a treatment with the inventive organic zinc (II) complex Zn(PA)₂ for 15 days.
Figure 5 shows the change in the body weight after a treatment with the inventive organic zinc (II) complex Zn(PA)₂ for 15 days.
Figure 6 shows the results of a glucose tolerance test after a treatment with an inventive organic zinc (II) complex for 15 days.

### Best Mode for Carrying Out the Invention

organic zinc (II) complexes according to the invention are preferably zinc (II) complexes of Zn(N₂O₂), Zn(O₄), Zn(O₂S₂), Zn(N₂S₂) or Zn(S₄) type.

The ligands in organic zinc (II) complexes according to the invention are pyridine ring-containing ligands

The pyridine ring-containing ligands are preferably ligands having a pyridine ring and a carboxyl group or its derivative such as pyridine carboxylic acids, pyridylacetic acids, nicotinamides, picolinamides and the like.

The preferred organic zinc (II) complexes according to the invention may for example be zinc (II) complexes of Zn(N₂O₂), Zn(O₄), Zn(O₂S₂), Zn(N₂S₂) or Zn(S₄) type comprising pyridine ring-containing ligands.

The preferred ligands of organic zinc (II) complexes of the invention are further detailed below.

The pyridine ring-containing ligands of organic zinc (II) complexes of the invention are represented by Formula (1): 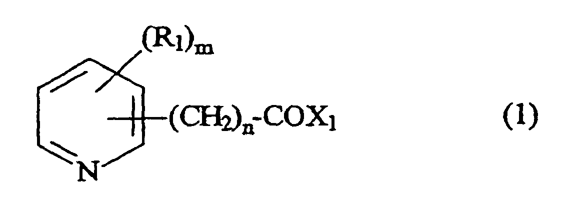
wherein:
m is I or more;
R₁ is hydrogen; unsubstituted hydrocarbon or hydrocarbon which is substituted by C₁₋₆ alkoxy, hydroxy, amino, a halogen, C₅₋₁₀ heterocyclyl or nitro; a halogen; hydroxyl; C₁₋₆ alkoxy; unsubstituted amino or amino which is substituted by hydrocarbon or hydrocarbon-derived acyl; unsubstituted C₅₋₁₀ heterocyclyl or C₅₋₁₀ heterocyclyl which is substituted by C₁₋₆ alkoxy, hydroxy, amino, a halogen, C₅₋₁₀ heterocyclyl or nitro; and, when R₁ occurs twice or more on the pyridine ring, the R₁ groups are selected independently;
X₁ is C₁₋₆ alkoxy; unsubstituted amino or amino which is substituted by C₁₋₆ alkyl; or hydroxyl; and
n is an integer from 0 to 5,

wherein a hydrocarbon is C₁₋₆ alkyl, C₂₋₆ alkenyl, C₂₋₆ alkynyl, C₆₋₁₀ cycloalkyl, C₆₋₁₀ cycloalkenyl or C₆₋₁₀ aryl.

A substituent R₁ in Formula (1) shown above is not limited particularly to those described above as long as insulin-like effects or hypoglycemic effects according to the invention is not affected adversely, and it may be bound in any position on the pyridine ring, and two or more substituents for example as R₁ groups may be bound on the pyridine ring.

A group -COX₁ denotes a carboxyl group or the derivatives, to which it is not limited as long as insulin-like effects or hypoglycemic effects according to the invention is not affected adversely. A group -(CH₂)ₙ-COX₁ may be bound in any position on the pyridine ring.

While n denotes an integer of 0 to 5 to which it is not limited as long as it enables a coordination with the center metal zinc and does not affect insulin-like effects or hypoglycemic effects of the invention adversely, it is preferably an integer of 0 to 5, more preferably 0 to 3, particularly 0 to 1.

The substituents R₁, on the pyridine, ring may be any of various organic residues as long as they do not affect an insulin-like effect or a hypoglycemic effect of the invention adversely, and those employed preferably be optionally substituted hydrocarbon groups, halogen atoms, hydroxyl group, alkoxy groups, optionally substituted amino groups, optionally substituted heterocyclic groups and the like.

The hydrocarbon groups may for example be lower alkyl groups, lower alkenyl groups, lower alkynyl groups, cycloalkyl groups, cycloalkenyl groups, aryl groups and the like.

The "lower alkyl group" employed in the invention may for example be a straight or branched alkyl group having 1 to 15, preferably 1 to 10, more preferably 1 to 6, particularly 1 to 4 carbon atoms, typically including methyl, ethyl, propyl, isoprogyl, butyl, isobutyl, s-butyl, t-propyl, pentyl, hexyl groups and the like. The "lower alkenyl group" may for example be a straight or branched alkenyl group having 2 to 15, preferably 2 to 10, more preferably 2 to 6 carbon atoms, typically including vinyl, allyl, butenyl, pentenyl groups and the like. The "lower alkynyl group" may for example be a straight or branched alkynyl group having 2 to 15, preferably 2 to 10, more preferably 2 to 6 carbon atoms, typically including ethynyl, propynyl, butynyl, pentynyl groups and the like.

The "cycloalkyl group" may for example be a monocyclic, polycyclic or fused cycloalkyl group having 3 to 30, preferably 5 to 20, more preferably 6 to 10 carbon atoms, and the "cycloalkenyl group" may for example be a cycloalkyl group mentioned above further containing one or more unsaturated groups such as a double bond.

The "aryl group" may for example be a monocyclic, polycyclic or fused aromatic hydrocarbon group having 6 to 30, preferably 6 to 20, more preferably 6 to 10 carbon atoms.

The "lower alkylene group" may for example be a straight or branched alkylene group having 1 to 15, preferably 1 to 10, more preferably 1 to 6, particularly 1 to 4 carbon atoms. When such a lower alkylene group is taken together with an adjacent atom to form a ring, one or more of the carbon atoms of the lower alkylene group may be replaced with an oxygen, nitrogen or sulfur atom.

The "hydrocarbon group" described above may further contain a substituent. Such a substituent may for example be a lower alkoxy group derived from a lower alkyl group described above, a hydroxy group, an amino group, a halogen atom, a heterocyclic group, a nitro group and the like.

The "halogen group" may for example be chlorine, bromine, iodine and the like.

The "alkoxy group" may for example be a lower alkoxy group derived from a lower alkyl group described above. The substituent on an "optionally substituted amino group" may for example be a hydrocarbon group described above and an acyl group derived from such a hydrocarbon group.

The "heterocyclic group" may for example be a saturated or unsaturated, monocyclic, polycyclic or fused ring having in its ring at least one nitrogen atom, oxygen atom or sulfur atom in which each ring is 5- to 20-membered, preferably 5-to 10-membered, more preferably 5- to 7-membered and which may be fused with a hydrocarbon group such as a cycloalkyl, cycloalkenyl or aryl group.

The heterocyclic group described above may further have a substituent, which may for example be a lower alkoxy group derived from the lower alkyl group described above, a hydroxy group, an amino group, a halogen atom, a heterocyclic group, a nitro group and the like.

The organic zinc (II) complexes of the invention can be produced by known methods (for example, US Patent No.5,219,847) or the methods according to such known methods. For example, the solution of an intended ligand is combined with the solution of a zinc salt to form an organic zinc (II) complex, which is then isolated as a product. While a preferred solvent is usually water, organic solvents or solvent mixtures may also be employed. The solution of a zinc salt is preferably the aqueous solution of an inorganic zinc such as zinc sulfate, zinc nitrate, zinc chloride and the like. It may sometimes be preferred to adjust the pH of the reaction mixture. The pH modifier may be the basic aqueous solution of sodium hydroxide, lithium hydroxide, barium hydroxide and the like.

More typically, examples described below can be followed.

The organic zinc (II) complexes of the invention have insulin-like effects or hypoglycemic effects as evidents also from experiments described below, and are useful as prophylactic or therapeutic agents against diabetes or hypertension.

Accordingly, the invention provides pharmaceutical compositions comprising organic zinc (II) complexes of the invention described above and pharmaceutically acceptable carrier.

In order to obtain the formulation, the organic zinc (II) complexes according to the invention are employed as active ingredients to form pharmaceutical formulations together with pharmaceutically acceptable carriers such as organic or inorganic, solid or liquid excipients suitable for oral, parenteral or topical administrations. Such pharmaceutical formulations may for example be capsule, tablet, syrup, granule, inhalant, suppository, liquid, lotion, suspension, emulsion, ointment, gel and the like. If necessary, any of the formulations described above may contain auxiliary agents, stabilizers, humectants or emulsifiers, buffering agents or other customary additives.

The invention also provides oral formulations comprising organic zinc (II) complexes of the invention. The inventive oral formulations are oral formulations comprising organic zinc (II) complexes having insulin-like effects or hypoglycemic effects. Accordingly, the invention provides a prophylactic or therapeutic agent against diabetes which can orally be administered. Those also provided are the use of organic zinc (II) complexes for producing prophylactic or therapeutic agents against diabetes which can orally be administered and methods for preventing or treating diabetes by means of oral administrations of therapeutically effective amounts of prophylactic or therapeutic agents against diabetes which can orally be administered.

While the therapeutically effective amount of organic zinc (II) complexes of the invention may vary depending on the ages and the condition of the patients to be treated, the dosage which give about 0.1 mg/patient or about 1000 mg/patient as mean single doses of the organic zinc (II) complexes of the invention may be given once to several times a day.

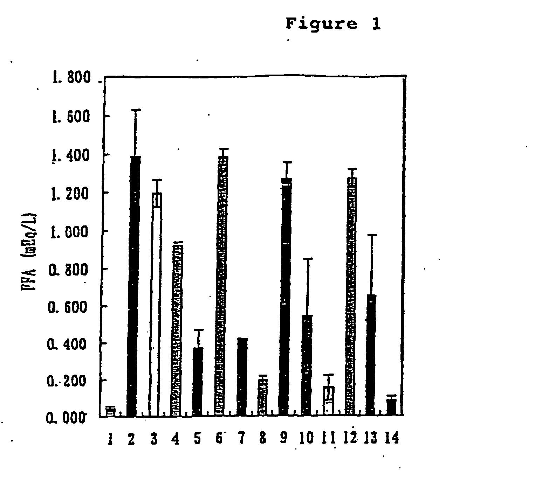

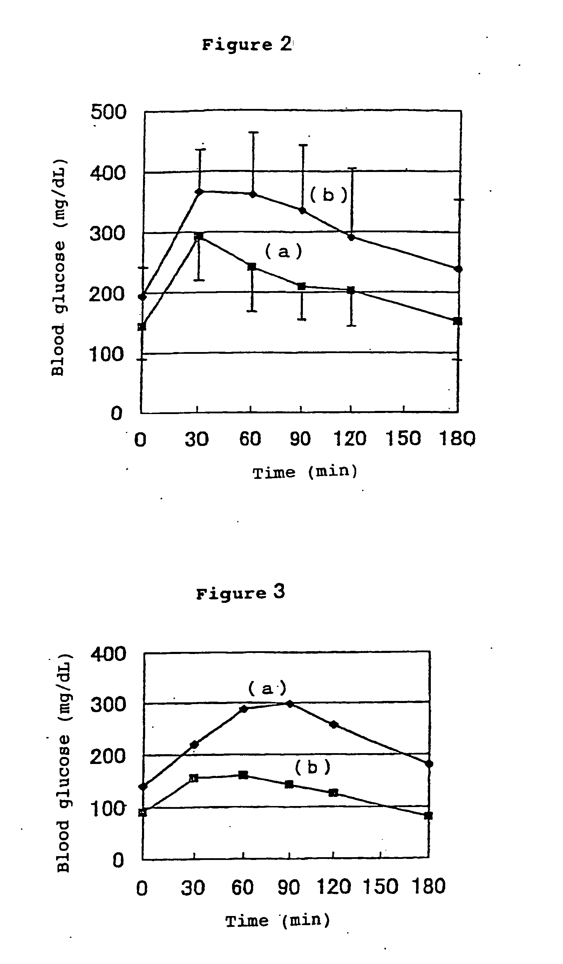

The results of the study on the inhibitory effect of organic zinc (II) complexes of the invention on the release of a fatty acid in rat fat cells are shown in Figure 1 and Figure 2.

Figure 1 shows the free fatty acid inhibiting effect observed when an organic zinc (II) complex of the invention was added to an epinephrine-stimulated rat adipocyte.

In Figure 1, the results are designated by 1 for a blank, 2 for a control, 3 to 5 for positive controls using oxovanadium sulfate (VOSO₄) and 6 to 14 for inventive compounds.

The results are designated by 6 to 8 for zinc (II) picolic acid complex [Zn(PA)₂(H₂O)₂], 9 to 11 for zinc (II) 6-methyl-picolic acid complex [Zn(MPA)₂(H₂O)] and 12 to 14 for zinc (II) pyridine-2-acetic acid complex [Zn(PAA)₂(H₂O)₂].

Each compound of 3 to14 was employed at 10⁻⁴M, 5 x 10⁻⁴M, 10⁻³M.

In Figure 1 , the "blank" designated by 1 indicates the free fatty acid (FAA) level due to the spontaneous release by cells, while the "control" designated by 2 indicates the free level due to the stimulation by epinephrine. The "VOSO₄" in 3 to 5 in Figure 1 means oxovanadium sulfate as a comparative example.

The IC₅₀(mM) values as the test substance concentrations capable of inhibiting the release of the fatty acid by 50 % calculated for inventive zinc (II) complexes based on the results described above and the results obtained similarly for other inventive zinc (II) complexes than those described above are shown in Table 1. The relative values on the bas is of the IC₅₀(mM) of "VOSO₄" being regarded as 1.00 mM are indicated in Table 1. 6-Methyl-picolinic acid complex [Zn(MPA)₂(H₂O)] and zinc (II) pyridine-2-acetic acid complex [Zn(PAA)₂(H₂O)₂] were determined in DMSO (dimethylsulfoxide), while other test substances were determined in physiological saline.

**Table 1**

| Complex | IC₅₀(mM) | | Remarks |
|---|---|---|---|
| | Glucose + | Glucose - | |
| trans-[Zn(PA)₂(H₂O)₂] | 0.50 | 0.57 | PA=Picolic acid |
| Zn(6MPA)₂(H₂O) | 0.39 | | 6MPA=6-Methylpicolic acid |
| Zn(3MPA)₂ | | 0.42 | 3HPA=3-methylpicolic acid |
| Zn(6BPA)₂ | | 0.45 | 6BPA=6-Bromopicolic acid |
| Zn(PAA)₂/2.4H₂O | 0.39 | | PAA=Pyridyl-2-acetic acid |
| Zn(Nic)₂/4H₂O | 0.82 | | Nic=Nicotinic acid |
| VOSO₄/5.4 or 2.3H₂O | 1.00 | 1.00 | VOSO₄=Oxovanadium sulfate |

As evident from these results, an organic zinc (II) complex of the invention exhibited a significant inhibitory effect on the release of a fatty acid from a rat adipocyte when compared with VOSO₄ and thus was proven to be excellent as a prophylactic or therapeutic agent against a diabetes or a hypertension.

Subsequently, KR-A^{y} mice as type II diabetes model animals and humans were subjected to the glucose tolerance tests to obtain blood glucose curves, which are shown in Figure 2 and Figure 3.

In Figure 2, (a) denotes a blood glucose curve obtained in a glucose tolerance test in mice each of which received 4 mg/kg of Zn(PA)₂(H₂O)₂ as an inventive zinc (II) complex once a day for 3 days and then fasted for 14 hours, and (b) denotes one in a control group in which the mice were not treated with the complex. In Figure 3, (a) denotes a blood glucose curve obtained in a glucose tolerance test in diabetes patients, while (b) denotes one in normal healthy volunteers.

Figure 3 shows the results of a glucose tolerance test employed in human diabetes tests [A.HAGURA, "NIPPON RINSHO (extra edition), Diabetes 3", in-all vol.728, p419 "KK NIPPONRINSHOSHA"], and reveals that a volunteer whose glucose metabolism was normal exhibited a slight increase in the blood glucose after an oral administration of glucose followed by a rapid decrease and then recovered the level before the administration after 180 minutes. On the other hand, a diabetes patient whose glucose metabolism was abnormal exhibited an increase in the blood glucose to the level approximately twice that in the normal healthy volunteers which was kept for a while and did not recover the level before the glucose administration even after 180 minutes.

On the contrary, a glucose tolerance test in KK-A^{y} mice revealed, as evident from Figure 2, that each mouse receiving 4 mg/kg of Zn(PA)₂(H₂O)₂ as an inventive zinc (II) complex once a day for 3 days and then fasting for 14 hours exhibited, after administering glucose, a reduced peak level of the blood glucose followed by a rapid decrease, as compared with the mice not receiving the complex and then recovered its almost normal blood glucose level after 180 minutes.

Based on the results described above, the type II diabetes was ameliorated in the mice receiving the inventive zinc (II) complex.

### Examples

The invention is further detailed in the following examples, which are not intended to restrict the invention.

### Example 1

### Bis(picolinate) zinc (II) complex [Zn(PA)₂(H₂O)₂] production

According to the method by Lumme et al (P.Lumme et al., Acta Chemica Scandinavica, 23, 3011-22 (1969)), picolic acid (10 mM) was dissolved in 10 ml of an aqueous solution of lithium hydroxide (10 mM). To this solution, an aqueous solution of zinc (II) sulfate (5 mM) was added dropwise in potions with stirring. After several days, the precipitation formed was recovered by a filtration, washed thoroughly with water, dried to obtain 3.26 g of the desired product as a white substance (yield 90 %).

The resultant trans-bis(picolinate) zinc (II) complex [Zn(PA)₂(H₂O)₂] had the structure shown below.

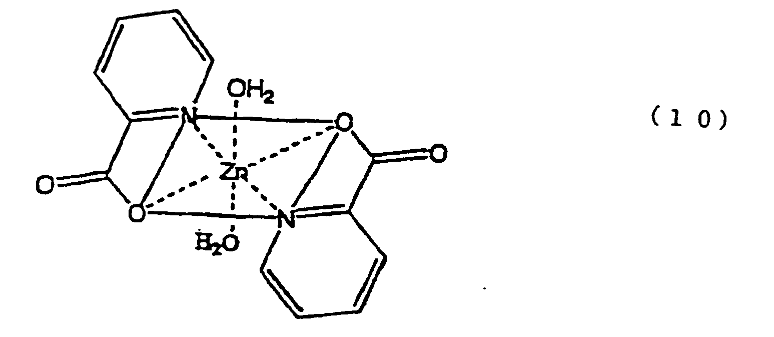

### Example 2

According to the method in Example 1, zinc (II) 6-methyl-picolinic acid complex [Zn(MPA)₂(H₂O)] was produced.

The resultant bis(6-methyl-picolinate) zinc(II) complex [Zn(MPA)₂(H₂O)] had the structure shown below.

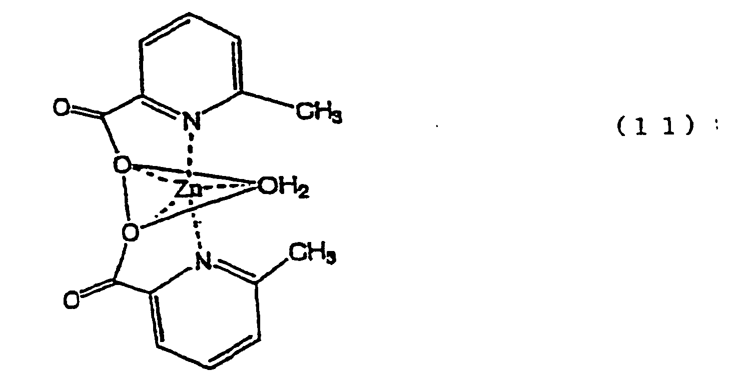

### Example 3

According to the method by Faure et al (P.R.Faure et al., Acta Cryst., B28, 811-5(1972)), bis(pyridine-2-acetate) zinc (II) complex [Zn(PAA)₂(H₂O)₂] was produced.

The resultant trans-bis(pyridine-2-acetate) zinc (II) complex [Zn(PAA)₂(H₂O)₂] had the structure shown below.

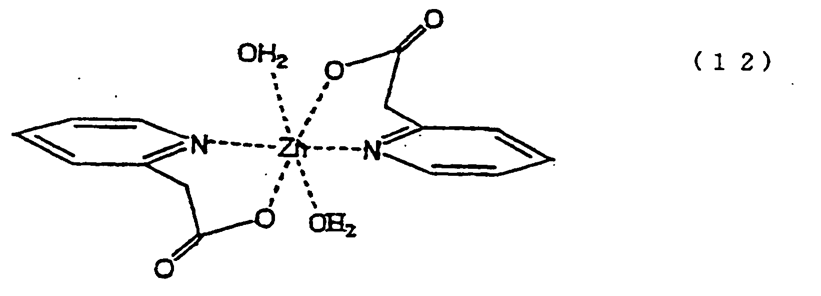

### Pharmacological test example 1

A pharmacological test was conducted as described below in accordance with the method reported in Biol.Pharm.Bull., 18, 719-725 (1995).

### (1) Preparation of rat adipocyte

A male Wistar rat weighing 200 g was exsanguinated under an anesthesia with ether, and a fat cell mass was isolated from a fat tissue around an epididymis according to the method of Road Bell (J. Biol. Chem., 239, 375(1964)). The fat cell mass was cut into pieces using scissors, and digested for 1 hour at 37°C in a KRB buffer solution (5 mM Glucose, 120 mM NaCl, 1.27 mM CaCl₂, 1.2 mM MgSO₄, 4.75 mM KCl, 1.2 mM KH₂PO₄ and 24 mM NaHCO₂; pH=7.4) supplemented with 20 mg of bovine serum albumin (BSA) and 2 mg of collagenase per ml. The fat cells were sieved through a nylon mesh (250 µm) whereby being made free from any undigested tissue, washed three times with the buffer similar to that described above except for containing no collagenase, and adjusted at 2.5 x 10⁶ cells/ml.

### (2) Effect of zinc (II) complex on rat adipocyte

The adipocyte separated as described above contained in a silicone-treated vial (2.5 x 10⁶ cells/ml) were preincubated for 0.5 hours at 37°C in 1 ml of a KRB buffer solution supplemented with 20 mg BSA/ml in the presence of VOSO₄ and each inventive organic Zn (II) complex at a varying concentration (10⁻⁴, 5x10⁻⁴, 10⁻³). Then the reaction mixture was combined with 10⁻⁵ M epinephrine and the resultant solution was incubated for 3 hours at 37°C. The reaction was quenched by cooling with ice, and the mixture was centrifuged for 10 minutes at 3000 rpm. The extracellular solution was examined for its free fatty acid (FFA) level using an NEFA kit whereby calculating an IC₅₀ value.

The results are shown in Figure 1 and Table 1.

Based on the results described above, each organic zinc (II) complex of the invention was proven to be capable of inhibiting the release of the fatty acid from the rat adipocyte significantly when compared with VOSO₄, thus being excellent as a drug for treating diabetes.

### Pharmacological test example 2

### (1) Methods

8-Week old KK-A^{y} mice were treated intraperitoneally once a day with a 5 % acacia solution in a control group (n=6) or with Zn(PA)₂ (n=6) dissolved in a 5 % acacia solution in the complex treatment groups.

The mice exhibiting blood glucose levels of 200 mg/d L or higher were treated at 4.5 mg Zn/kg and the mice of 150 mg/d L or higher but less than 200 mg/dL at 2.5 mg Zn/kg, while the mice of less than 150 mg/dL were not treated. In the control group, 0.5 ml of the acacia solution was given.

The onset of a diabetes was ensured on the basis of a mean blood glucose level immediately before the treatment of 450 mg/dL or higher and a mean body weight of 35 g or higher.

The blood glucose was determined using a simplified blood sugar tester (GLUCOCARD, manufactured by KYOTO DAIICH KAGAKU).

### (2) Results and discussion

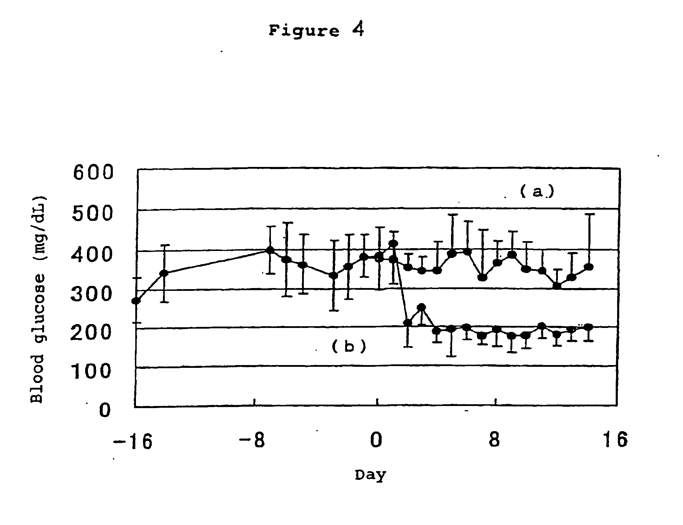

The blood glucose change after the treatment with Zn(PA)₂ for 15 days is shown in Figure 4.

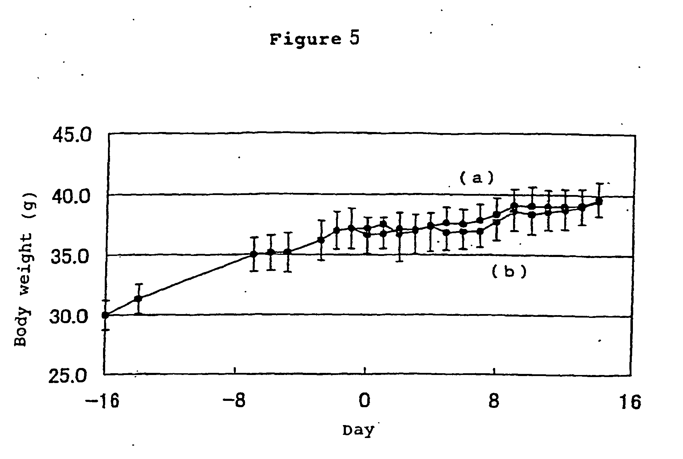

The body weight change after the treatment with Zn(PA)₂ for 15 days is shown in Figure 5.

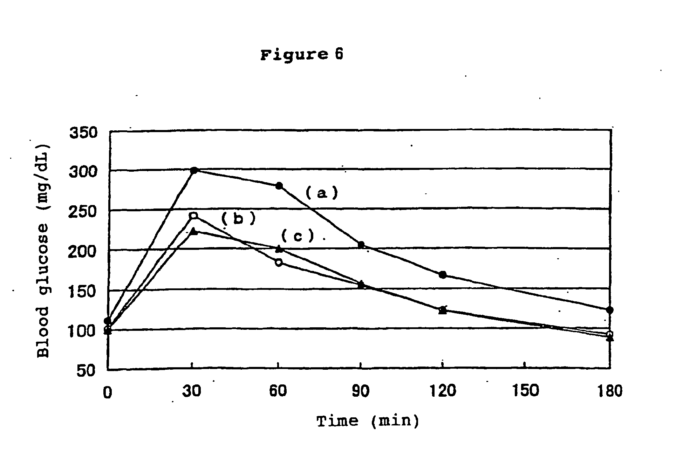

The results of a glucose tolerance test after a treatment with a complex for 15 days are shown in Figure 6. (glucose tolerance test: A mouse was fasted for 13 hours and then administered orally with glucose at 1 g/kg, and then the blood glucose levels were determined at certain time intervals.)

In Figures 4 to 6, the results are designated by (a) in the control group, by (b) in the group treated with the complex Zn(PA)₂.

The zinc complex was highly effective in normalizing the blood glucose level as compared with the control group (Figure 4).

The weight loss, which is an index of side effects, was not observed in most of the animals treated with Zn(PA)₂(Figure 5).

Also using Zn(PA)₂ complex, the glucose tolerance test was conducted after the administration of each complex for 15 days, and the results revealed an amelioration of the diabetes conditions when compared with the diabetes mice (control mice) (Figure 6).

### Industrial Applicability

The organic zinc (II) complexes of the invention are highly stable and have fat-soluble insulin-like effects and hypoglycemic effects. Accordingly, the organic zinc (II) complexes of the invention are useful as prophylactic or therapeutic agent against glucose tolerance impairment, diabetes (such as type II diabetes), insulin resistant syndrome (such as insulin receptor dysfunction), polycystic ovary syndrome, hyperlipidemia, atherosclerosis, cardiovascular disease (such as angina pectris, heart failure), hyperglycemia or hypertension, or angina pectris, hypertension, pulmonary hypertension, congestive heart failure, diabetic complication (such as diabetic gangrene, diabetic arthropathy, diabetic glomeruloscrelosis, diabetic dermopathy, diabetic neuropathy, diabetic cataract, diabetic retinophathy).

## Claims

1. An organic zinc (II) complex, comprising zinc and a ligand as defined in Formula (1), for use in a method of treatment on the human or animal body:
a carboxylic acid derivative of pyridine of Formula (1): 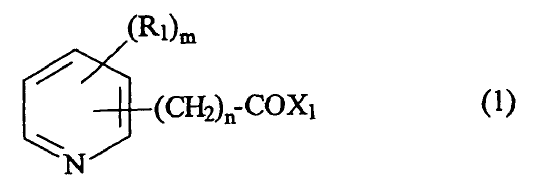
wherein:
m is 1 or more;
R₁ is hydrogen; unsubstituted hydrocarbon or hydrocarbon which is substituted by C₁₋₆ alkoxy, hydroxy, amino, a halogen, C₅₋₁₀ heterocyclyl or nitro; a halogen; hydroxyl; C₁₋₆ alkoxy; unsubstituted amino or amino which is substituted by hydrocarbon or hydrocarbon-derived acyl; unsubstituted C₅₋₁₀ heterocyclyl or C₅₋₁₀ heterocyclyl which is substituted by C₁₋₆ alkoxy, hydroxy, amino, a halogen, C₅₋₁₀ heterocyclyl or nitro; and, when R₁ occurs twice or more on the pyridine ring, the R₁ groups are selected independently;
X₁ is C₁₋₆ alkoxy; unsubstituted amino or amino which is substituted by C₁₋₆ alkyl; or hydroxyl; and
n is an integer from 0 to 5,
wherein a hydrocarbon is C₁₋₆ alkyl, C₂₋₆ alkenyl, C₂₋₆ alkynyl, C₆₋₁₀ cycloalkyl, C₆₋₁₀ cycloalkenyl or C₆₋₁₀ aryl.

2. A complex according to claim 1 wherein the organic zinc (II) complexes are zinc (II) complexes are complexes of the type Zn(N₂O₃), Zn(O₄), Zn(O₂S₂), Zn(N₂S₂) or Zn(S ₄).

3. Use of an organic zinc (II) complex as defined in either claim 1 or claim 2 in the manufacture of a medicament for the treatment of diabetes.

4. Use according to claim 3 wherein the medicament is in a form suitable for oral administration.

5. Use according to claim 3 or claim 4 wherein the medicament displays insulin-like effects.

6. Use according to any one of claims 3 to 5 wherein the medicament is a hypoglycemic agent.

## Patentansprüche

1. Organischer Zink-(II)-Komplex, umfassend Zink und einen Liganden, wie in Formel (1) definiert, zur Verwendung bei einer Behandlungsmethode am menschlichen oder tierischen Körper:
ein Carbonsäure-Derivat von Pyridin der Formel (1): 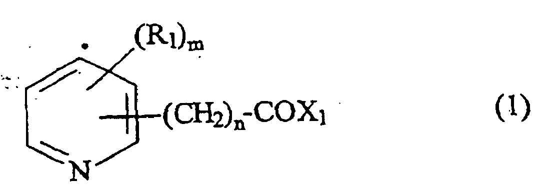
worin:
m 1 oder mehr ist;
R₁ Wasserstoff, unsubstituierter Kohlenwasserstoff oder Kohlenwasserstoff ist, der substituiert ist durch C₁₋₆-Alkoxy, Hydroxy, Amino, ein Halogen, C₅₋₁₀₋Heterocyclyl oder Nitro; ein Halogen; Hydroxyl; C₁₋₆-Alkoxy; unsbustituiertes Amino oder Amino, das substituiert ist durch Kohlenwasserstoff oder Kohlenwasserstoff-abgeleitetes Acyl; unsubstituiertes C₅₋₁₀-Heterocyclyl oder C₅₋₁₀-Heterocyclyl, das substituiert ist durch C₁₋₆-Alkoxy, Hydroxy, Amino, ein Halogen, C₅₋₁₀-Heterocyclyl oder Nitro; und worin, wenn R₁ zweifach oder mehrfach am Pyridinring vorkommt, die R₁-Gruppen unabhängig ausgewählt sind;
X₁ C₁₋₆-Alkoxy; unsbustituiertes Amino oder Amino, das substituiert ist durch C₁₋₆₋Alkyl; oder Hydroxyl ist; und
n eine ganze Zahl von 0 bis 5 ist;
worin ein Kohlenwasserstoff C₁₋₆-Alkyl, C₂₋₆-Alkenyl, C₂₋₆-Alkynyl, C₆₋₁₀-Cycloalkyl, C₆₋₁₀-Cycloalkenyl oder C₆₋₁₀-Aryl ist.

2. Komplex nach Anspruch 1, wobei die organischen Zink-(II)-Komplexe Zink-(II)-Komplexe des Typs Zn(N₂O₂), Zn(O₄), Zn(O₂S₂), Zn(N₂S₂) oder Zn(S₄) sind.

3. Verwendung eines organischen Zink-(II)-Komplexes, wie entweder in Anspruch 1 oder Anspruch 2 definiert, in der Herstellung eines Medikaments für die Behandlung von Diabetes.

4. Verwendung nach Anspruch 3, wobei das Medikament in einer Form vorliegt, die für die orale Verabreichung geeignet ist.

5. Verwendung nach Anspruch 3 oder Anspruch 4, wobei das Medikament Insulinartige Wirkungen zeigt.

6. Verwendung nach einem der Ansprüche 3 bis 5, wobei das Medikament ein hypoglykämisches Mittel ist.

## Revendications

1. Complexe organique du zinc (II) comprenant du zinc et un ligand tel que défini dans la formule (1), pour utilisation dans un procédé de traitement de l'organisme humain ou animal :
un dérivé acide carboxylique de la pyridine de formule (I) : 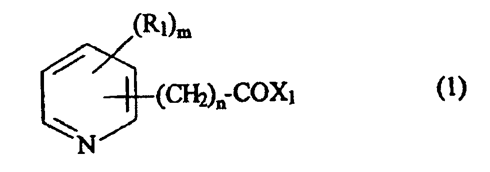
dans laquelle
m vaut 1 ou plus ;
R₁ est un atome d'hydrogène ; un radical hydrocarboné non substitué, ou hydrocarboné à substitution alcoxy en C₁₋₆, hydroxy, amino, halogéno, hétérocyclyle en C₅₋₁₀ ou nitro ; un atome d'halogène, le groupe hydroxyle ; un groupe alcoxy en C₁₋₆ ; le groupe amino non substitué, ou amino substitué par des substituants hydrocarbonés ou acyle dérivés d'hydrocarbures ; un radical hétérocyclyle en C₅₋₁₀ non substitué, ou hétérocyclyle en C₅₋₁₀ à substitution alcoxy en C₁₋₆, hydroxy, amino, halogéno, hétérocyclyle en C₅₋₁₀ ou nitro ; et, quand R₁ apparaît au moins deux fois dans le noyau pyridine, les groupes R₁ sont choisis d'une manière indépendante ;
X₁ est un groupe alcoxy en C₁₋₆ ; amino non substitué ou amino à substitution alkyle en C₁₋₆ ; ou hydroxyle ; et
n est un entier de 0 à 5,
où le radical hydrocarboné est un groupe alkyle en C₁₋₆, alcényle en C₂₋₆, alcynyle en C₂₋₆, cycloalkyle en C₆₋₁₀, cycloalcényle en C₆₋₁₀ ou aryle en C₆₋₁₀.

2. Complexe selon la revendication 1, dans lequel les complexes organiques du zinc (II) sont des complexes du zinc(II) du type Zn(N₂O₂), Zn(O₄), Zn(O₂S₂), Zn(N₂S₂) ou Zn(S₄).

3. Utilisation d'un complexe organique du zinc(II) selon l'une ou l'autre des revendications 1 ou 2 pour préparer un médicament destiné au traitement du diabète.

4. Utilisation selon la revendication 3, pour laquelle le médicament se présente sous une forme convenant à une administration orale.

5. Utilisation selon la revendication 3 ou 4, pour laquelle le médicament présente des effet insulinoïdes.

6. Utilisation selon l'une quelconque des revendications 3 à 5 pour laquelle le médicament est un agent hypoglycémique.
